# FASCICULE DE BREVET EUROPEEN

(11) **EP 3 468 451 B1**
(45) Date de publication et mention de la délivrance du brevet: **10.04.2024**
(21) Numéro de dépôt: 17734789.5
(22) Date de dépôt: 08.06.2017
(51) Int. Cl.: A61B 5/00

(54) **SURVEILLANCE DE LA QUALITÉ DU SOMMEIL D'UN BÉBÉ PAR COMBINAISON DE DONNÉES MULTIMODALES INCLUANT LA PHOTOPLÉTHYSMOGRAPHIE**
ÜBERWACHUNG DER QUALITÄT DES SCHLAFS EINER KLEINKINDES DURCH KOMBINATION VON MULTIMODALEN DATEN EINSCHLIESSLICH PHOTOPLETHYSMOGRAPHIE
MONITORING OF THE QUALITY OF THE SLEEP OF A BABY BY COMBINATION OF MULTIMODAL DATA INCLUDING PHOTOPLETHYSMOGRAPHY

(30) Priorité: 10.06.2016 FR 1655339
(43) Date de publication de la demande: 17.04.2019
(73) Titulaire: Université de Bourgogne, 21078 Dijon Cedex (FR)
(72) Inventeur: BENEZETH, Yannick, 21000 Dijon (FR); BOBBIA, Serge, 21000 Dijon (FR); DUBOIS, Julien, 21800 Quétigny (FR); MACWAN, Richard, 21000 Dijon (FR); MANSOURI, Alamin, 89000 Auxerre (FR)
(74) Mandataire: Novagraaf Technologies
(86) Numéro de dépôt international: PCT/FR2017/051443
(87) Numéro de publication internationale: WO 2017/212174

(56) Documents cités:
- US-A1- 2014 163 343
- US-A1- 2014 275 832
- US-A1- 2014 276 089
- US-A1- 2015 238 137

## Description

### DOMAINE DE L'INVENTION

La présente invention concerne la surveillance du sommeil d'un être vivant, notamment d'un bébé humain. Plus précisément, elle concerne un procédé et un dispositif pour la détermination automatique d'informations représentatives de la qualité dudit sommeil.

### CONTEXTE DE L'INVENTION

La surveillance du sommeil d'un être vivant peut avoir différentes applications. Dans le cas de bébé humain, et essentiellement de nourrissons, cette problématique peut revêtir des enjeux vitaux et cruciaux. Automatiser ce processus permet non seulement de libérer le personnel d'une tache fastidieuse, mais aussi d'améliorer la qualité de la surveillance d'une part en évitant les sous-détections par des manquements de l'attention du personnel surveillant mais aussi par la prise en compte de paramètres inaccessibles à un surveillant humain. En outre, une surveillance manuelle ne peut permettre une quantification précise permettant d'effectuer des comparaisons, des statistiques, etc.

Différents mécanismes ont été proposés pour permettre la détermination automatique d'informations représentatives de la qualité du sommeil d'un être humain.

A titre d'exemple, la demande de brevet US2011/0311119 décrit un mécanisme de détection automatique de signes vitaux à partir d'un flux vidéo issu d'une caméra pointée sur un être vivant. Une telle approche est toutefois tributaire de la présence d'une caméra vidéo et de la qualité du flux vidéo et, donc, des conditions de prise de vue. Ainsi, dans une pièce trop sombre, ou en cas de prise de vue en contre-jour, la qualité du flux vidéo risque ne pas être suffisant pour une bonne analyse et peut générer des mesures de mauvaise qualité, incomplète ou erronées.

Une autre contribution est décrite dans la demande de brevet US2014/276089. Mais celle-ci se base sur l'analyse d'un certaine nombre de zones (front...) fixées *a priori* comme devant être suivis, ce qui engendre une détection moins précise et erratique dans le cas où la zone serait non visible.

D'autres contributions sont formées par les demandes US2014/275832, US2014/163343 et US2015/238137, mais la qualité de la détection peut ne pas être suffisante ou satisfaisante.

Il est donc important de proposer des améliorations aux solutions de l'état de la technique.

### RÉSUMÉ DE L'INVENTION

Le but de la présente invention est de fournir une mesure de la qualité du sommeil d'un être vivant, préférentiellement d'un bébé, palliant au moins partiellement les inconvénients précités.

Une contrainte supplémentaire pour l'estimation de la qualité du sommeil d'un bébé est que le procédé doit se faire sans contact, et que certaines mesures acceptables pour un adulte peuvent ne pas l'être pour un bébé ou enfant en bas âge. L'invention vise principalement des enfants en très bas âge en évitant les types de mesure qui ne leurs sont pas acceptable, mais l'invention est également utilisable pour des enfants d'âge plus élevé, voire pour des adultes.

A cette fin, la présente invention propose un procédé de détermination automatique d'une information représentative de la qualité du sommeil d'un être vivant, sans contact avec ledit être vivant, à partir d'un ensemble de flux de données relatives audit être vivant, issus d'au moins deux capteurs, au moins un desdits capteurs étant une caméra vidéo filmant ledit être vivant et fournissant un flux correspondant à un paramètre physiologique dudit être vivant, établi par photopléthysmographie, ledit procédé consistant à
- établir des flux de mesures à partir desdits flux de données ;
- décomposer le flux établi par photopléthysmographie en régions d'intérêt, et établir une somme pondérée par une pulsatilité mesurée pour chaque région, des signaux de photopléthysmographie établi pour chaque région
- combiner lesdits flux de mesures pour fournir ladite information, en en prenant en compte une estimation de la qualité desdits flux de données.

Suivant des modes de réalisation préférés, l'invention comprend une ou plusieurs des caractéristiques suivantes qui peuvent être utilisées séparément ou en combinaison partielle entre elles ou en combinaison totale entre elles :
- ledit flux de mesures établi par photopléthysmographie est un rythme cardiaque ;
- ledit flux de mesures établi par photopléthysmographie est un rythme respiratoire ;
- lesdits flux de mesures représentent des données environnementales, des données liées à l'activité dudit être vivant, et des données physiologiques ;
- la mesure cardiaque réalisée par photopléthysmographie est améliorée par l'utilisation d'un modèle de la forme du signal PPG comme information a priori pour aider la convergence de la technique de séparation de sources ;
- un flux de mesures est un rythme respiratoire établi par analyse des mouvements de la cage thoracique dudit être vivant à partir d'un flux vidéo généré par ladite caméra vidéo ;
- la combinaison des flux de mesures est réalisée en estimant la fiabilité des flux de mesures par statistique bayésienne.

Un autre objet de l'invention est un dispositif pour détermination automatique d'une information représentative de la qualité du sommeil d'un être vivant, sans contact avec ledit être vivant, à partir d'un ensemble de flux de données relatives audit être vivant, issus d'au moins deux capteurs, au moins un desdits capteurs étant une caméra vidéo filmant ledit être vivant et fournissant un flux correspondant à une donnée physiologique dudit être vivant, établi par photopléthysmographie, ledit dispositif comportant des moyens pour :
- établir des flux de mesures à partir desdits flux de données ;
- décomposer le flux établi par photopléthysmographie en régions d'intérêt, et établir une somme pondérée par une pulsatilité mesurée pour chaque région, des signaux de photopléthysmographie établi pour chaque région
- combiner lesdits flux de mesures pour fournir ladite information, en en prenant en compte une estimation de la qualité desdits flux de données.

Encore un autre objet de l'invention est un système portant un dispositif tel que précédemment défini et une caméra vidéo pour filmer ledit être vivant, dans lequel ledit dispositif est prévu pour recevoir au moins un flux de données issu de ladite caméra vidéo.

D'autres caractéristiques et avantages de l'invention apparaîtront à la lecture de la description qui suit d'un mode de réalisation préféré de l'invention, donnée à titre d'exemple et en référence aux dessins annexés.

### BRÈVE DESCRIPTION DES DESSINS

La figure 1 représente schématiquement un exemple d'architecture fonctionnelle d'un dispositif conforme à un mode de réalisation de l'invention.
Les figures 2a et 2b schématise le principe de segmentation par le vivant mis en oeuvre dans un mode réalisation de l'invention.
La figure 3 illustre le principe de séparation des sources sous contrainte, utilisé dans un mode de réalisation de l'invention.
La figure 4 schématise une mise en oeuvre pratique possible du dispositif selon l'invention sur un site.

### DESCRIPTION DÉTAILLÉE DE L'INVENTION

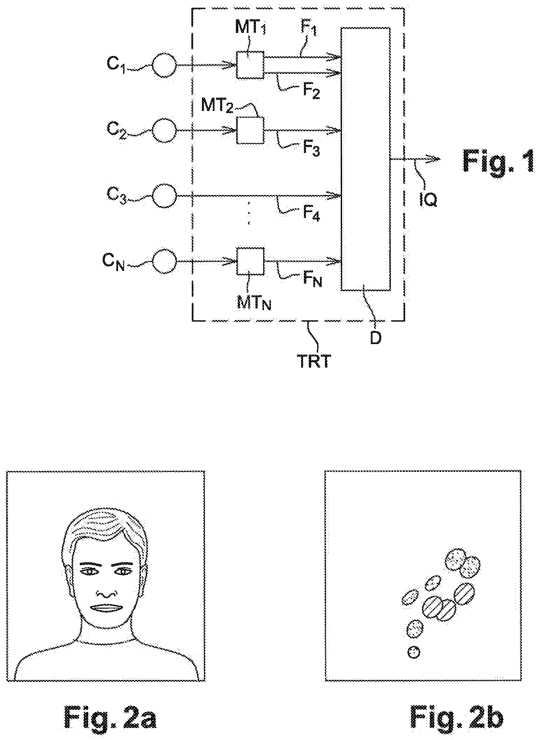

Ainsi qu'illustré par la figure 1, l'invention repose sur la prise en compte d'une combinaison de flux de données F₁, F₂, F₃... F_{N} issus de capteurs C₁, C₂, C₃...C_{M}, afin de de déterminer une information IQ sur la qualité du sommeil d'un être vivant, préférentiellement d'un bébé.

Une contrainte supplémentaire pour l'estimation de la qualité du sommeil d'un bébé est que le procédé doit se faire sans contact, et que certaines mesures acceptables pour un adulte peuvent ne pas l'être pour un bébé ou enfant en bas âge. L'invention vise principalement des enfants en très bas âge en évitant les types de mesure qui ne leurs sont pas acceptable, mais l'invention est également utilisable pour des enfants d'âge plus élevé, voire pour des adultes.

Dans le mode de réalisation de l'invention illustré par la figure 1, les capteurs C₁, C₂, C₃...C_{M} sont situés à proximité du bébé dont on veut surveiller le sommeil. Il peut s'agir de capteurs de son (microphone), de capteurs environnementaux (température, hydrométrie...), d'une caméra vidéo, etc.

Ces différents capteurs peuvent transmettre des données brutes à des modules de traitements, MT₁, MT₂... MT_{M}, dont le rôle est de fournir des flux de données F₁, F₂, F₃, F_{N} exploitables par le dispositif D en charge de la détermination de l'information IQ sur la qualité du sommeil. D'une façon générale, le nombre N de flux de données disponible peut être supérieur au nombre M de capteurs.

Un même module de traitement peut fournir plusieurs flux de données : par exemple, les données brutes issues d'une caméra vidéo C₁ peuvent fournir un ensemble de flux de données F₁, F₂ représentant plusieurs paramètres physiologiques. Il sera vu ultérieurement des cas concrets de tels flux de données.

Des capteurs C₃ peuvent également fournir des flux de données F₄ directement exploitables par le dispositif D.

Le dispositif D et les modules de traitement peuvent faire parties d'une même unité de traitement TRT qui peut être co-localisée ou répartie sur plusieurs machines.

Selon l'invention, au moins deux capteurs sont utilisés, afin de permettre une diversité du type de données et d'ainsi capturer une meilleure image globale de la qualité du sommeil. L'invention repose sur l'agrégation de flux de données issus d'au moins deux capteurs différents.

Selon un mode de réalisation préférentiel de l'invention, au moins un des capteurs est une caméra vidéo. Il peut s'agir d'une simple web-cam.

Le dispositif D est apte à établir des flux de mesures à partir des flux de données reçus. Par exemple, à partir d'un flux vidéo, différentes mesures peuvent être établies.

L'invention permet d'agréger des flux de données de natures différentes, permettant l'établissement de mesures multimodales, par exemple des mesures liées à l'environnement et des mesures correspondant à un paramètre physiologiques du bébé ou lié à son activité.

Le dispositif D peut ensuite combiner ces flux de mesures pour fournir l'information IQ sur la qualité du sommeil, en fonction d'une estimation de la qualité des flux de données.

Ainsi, le procédé selon l'invention peut s'adapter de façon dynamique aux conditions dans lesquelles se trouve le bébé et qui influent sur la qualité des données fournies par les capteurs. Selon un mode de réalisation de l'invention, les flux de mesures sont combinés en fonction d'une estimation de la fiabilité de chaque flux de mesures (ou « modalité »). Cette démarche peut par exemple être décrite en statistique Bayésienne par l'estimation de la probabilité maximum a posteriori (MAP, pour *"maximum a posterior probability* » en langue anglaise) qui permet d'incorporer une distribution a priori dépendante de la fiabilité que l'on peut accorder à une modalité à un instant donné. Par exemple, une fluctuation de la luminosité de la pièce peut rendre un algorithme de traitement du flux vidéo plus ou moins efficace, et le dispositif D peut alors pondérer l'importance accordée aux flux de données F₁, F₂ issus du capteur vidéo C₁ pour déterminer l'information IQ.

Il a été évoqué que des données environnementales peuvent être fournies par des capteurs de température et d'hygrométrie.

Des flux de mesures peuvent être représentatifs de l'activité du bébé. Ils peuvent être fournis par le volume sonore mesuré par un simple microphone. Ils peuvent être également fournis par le flux vidéo, par exemple en estimant une agitation du bébé en calculant pour chaque paire de trames vidéo la somme de la magnitude de tous les vecteurs du flot optique calculés pour tous les pixels. Cette valeur est ensuite normalisée par le nombre de pixels dans l'image. Ainsi, plus le bébé est agité et plus importante sera cette somme.

Ces deux mesures permettent ainsi de quantifier par des modalités complémentaires l'agitation du bébé.

Comme il a été évoqué précédemment, les données physiologiques du bébé sont mesurées sans contact. En utilisant des techniques de traitement du signal et de la vidéo, il est possible d'analyser les données fournies par le capteur vidéo C₁ pour extraire par exemple des mesures relatives au rythme cardiaque et au rythme respiratoire du bébé.

Un mode de réalisation préférentiel consiste à établir ce rythme cardiaque (ainsi que le rythme respiratoire) par photopléthysmographie à distance, à partir du flux vidéo.

La photopléthysmographie (PPG) est une technique de mesure optique permettant de déceler des changements de volumes sanguins dans des tissus vivants. Le principe repose sur la détection des variations de l'intensité lumineuse sur une zone de peau. La zone de la peau à mesurer peut être illuminée, mais dans le cadre de l'invention la lumière ambiante peut être utilisée. Une source de lumière infra-rouge dans le cas de surveillance nocturne peut être intégrée au système, et permet d'envisager une mesure quel que soit l'instant de la journée. En fonction du degré d'illumination, le flux de données fourni par la photopléthysmographie pourra ou non être utilisé, ainsi que précédemment évoqué, et éventuellement affecté d'un degré de confiance.

Les variations d'intensité lumineuse des zones de peau, mesurées par la caméra sont synchrones avec les battements du coeur. Le traitement consiste donc à déterminer les fréquences principales de ces variations et d'en déduire le rythme cardiaque et/ou respiratoire du bébé.

Les techniques de photopléthysmographie à distance sont connues en soi dans l'état de la technique. A titre d'exemple, on peut citer l'article « Unsupervised Subject Detection via Remote-PPG » de Wenjin Wang, Sander Stujk et Gérard de Haan, in IEEE Transactions on Biomédical Engineering, 2015, ou bien la demande de brevet WO2013027027 intitulée « *Remote monitoring of Vital Signs* »*.*

Les techniques de photopléthysmographie (PPG) en général sont plus abondamment décrites, notamment sur Wikipédia :

### https://en.wikipedia.org/wiki/Photoplethysmogram

Dans le cadre de l'invention, la technique de photopléthysmographie à distance (r-PPG pour « remote-PPG ») est utilisée sur des signaux vidéos issus d'une caméra vidéo.

Une des difficultés à résoudre est que le rapport signal à bruit est très faible.

Préférentiellement, le flux vidéo est décomposé en plusieurs régions d'intérêt. Ces régions peuvent être, par exemple, un ensemble de superpixels définis comme un ensemble de pixels contigus possédant les mêmes propriétés. Les régions de chaque image sont ensuite simplement mises en correspondance dans le temps.

Sur chaque région d'intérêt, une moyenne spatiale des valeurs des pixels est appliquée sur chaque trame afin de construire un signal à deux dimensions (canaux et temps). Les valeurs des pixels peuvent être considérées comme un vecteur à trois dimensions, typiquement RVB (Rouge - Vert - Bleu).

Les signaux de chaque région sont ensuite combinés par une somme pondérée où les poids sont déterminés par un critère de pulsatilité dans chaque région. Ainsi, les régions présentant un signal de bonne qualité auront un impact plus important que celles pour lesquelles le signal est bruité ou correspond à une zone de l'arrière-plan. La sélection des régions pertinentes est implicite, c'est-à-dire que les zones de peau contribuent significativement plus que les zones de l'arrière-plan sur le signal final. Cette méthode peut être qualifiée de « segmentation par le vivant ».

Cette segmentation par le vivant est illustrée par les figures 2a et 2b. La figure 2a représente une image d'un flot vidéo, représentant un portrait d'une personne. La figure 2b illustre les différentes régions extraites sur cette trame et leurs différentes contributions sur le signal final : les régions avec une texture pointillée correspondent à des contributions faibles, tandis que les régions hachurées correspondent à des contributions fortes. Le fond correspond à une contribution nulle ou proche de zéro.

Cette segmentation par le vivant permet notamment de s'affranchir d'étapes préalables de détection d'éléments et de suivi de ces éléments au cours du flux vidéo (les éléments pouvant être des zones de peau, le visage, les mains...) puisque de façon inhérente au procédé, la contribution de ces régions au signal PPG global est plus importante que les autres régions.

Lors de la combinaison des signaux, on peut prendre en compte les décalages de phase dû au temps nécessaire à l'onde pulsatile pour se propager à travers le réseau sanguin. Ce temps est typiquement appelé « Pulse Transit Time » (PTT) en anglais. Pour cela, on supprime le décalage de phase entre les différents signaux de chaque région.

Le signal à deux dimensions (RVB et temps) est composé d'un mélange de plusieurs sources dont une représente le signal photopléthysmographique que l'on souhaite extraire et les autres correspondent à des signaux non pertinents (variation de lumière, mouvement, etc.). Une technique de séparation de sources est appliquée pour isoler le signal PPG. Nous n'employons pas une séparation de sources aveugle (BSS, pour « *Blind Source Séparation* » en anglais) mais nous ajoutons comme information a priori à la fonction d'optimisation, la forme du signal PPG recherché afin de faciliter la convergence de l'algorithme. Le signal PPG a donc été modélisé et ce modèle est utilisé pour guider la séparation de sources.

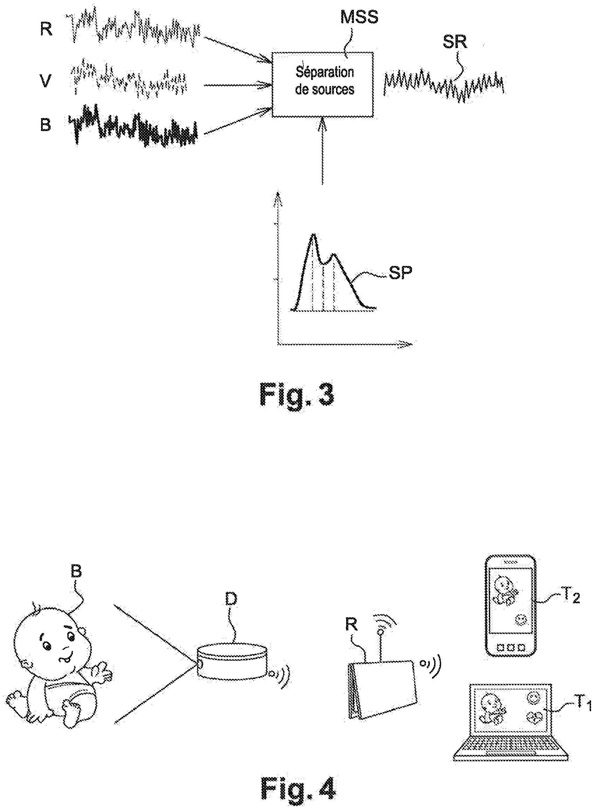

La figure 3 illustre la séparation de sources sous contraintes. Les trois signaux R, V, B correspondant aux composantes classiques « rouge, vert, bleu » d'un signal vidéo composite sont traitées par le module fonctionnel de séparation de sources MSS, en utilisant le signal photopléthysmographique recherché SP. Ce signal recherché, utilisé comme « a priori » permet alors de déterminer le signal résultant SR fourni par ce module de séparation de sources MSS.

Une analyse temps-fréquence peut ensuite être mise en oeuvre afin d'obtenir par exemple le rythme cardiaque à chaque instant sur le principe d'une fenêtre glissante en sélectionnant les fréquences principales.

En plus des variations dans la modulation de la lumière dues au cycle cardiaque, le signal PPG contient également une modulation due au cycle respiratoire. A partir du signal PPG estimé par la méthode qui vient d'être exposée, on peut donc également obtenir le rythme respiratoire en analysant le signal à différentes fréquences.

Si les conditions d'éclairage sont mauvaises (pièce trop sombre, par exemple, ou contre-jour, ou peau insuffisamment visible...), les faibles variations colorimétriques capturées par la caméra vidéo peuvent être insuffisante pour établir une bonne estimation du rythme cardiaque et/ou du rythme respiratoire par photopléthysmographie.

Dans ce cas d'autres techniques peuvent être utilisées. Le choix d'une technique peut être effectuée par estimation de la qualité des flux de données, ainsi que précédemment décrit.

Par exemple, une estimation du rythme respiratoire peut être établie analyse des mouvements de la cage thoracique du bébé, à partir d'un flux vidéo généré par ladite caméra vidéo.

Différentes techniques peuvent être mises en oeuvre pour ce faire. Par exemple, on peut analyser les déplacements dans l'image des pyramides de valeurs complexes (« *complex steerable pyramids* » en langue anglaise), tel que cela a été proposé dans l'article « Phase-based Video Motion Processing » de Neal Wadhwa et al., in SIGGRAPH 2013.

L'ensemble des flux de mesures peuvent être combiné pour fournir une information IQ sur la qualité du sommeil du bébé, et ainsi permettre la surveillance de la qualité du sommeil.

Ainsi, le rythme cardiaque est révélateur de la qualité du sommeil, et peut être corrélé avec d'autres données environnementales pour établir des informations plus complètes. Ainsi, une augmentation du rythme cardiaque du bébé peut trouver une cause probable si corrélé avec une augmentation substantielle de la température de la pièce. La mise en correspondance de ces deux données permet de comprendre la situation et d'intervenir de façon adéquate.

Ces informations peuvent être simplement affichées sur un moniteur, ou transmises à un dispositif mobile de télécommunication, comme un « smartphone » ou une tablette numérique. Elles peuvent être mémorisées afin de permettre l'établissement d'un historique. Elles peuvent aussi être mises en correspondance avec des seuils permettant le déclenchement d'alertes en cas de franchissement.

La figure 4 schématise une mise en oeuvre pratique possible. Un dispositif D fournit un ensemble de flux de données relatif au bébé B à un ou plusieurs terminaux T1, T2. Ces flux de données peuvent être transmis par un réseau local radio, tel un réseau WIFI, et par exemple par le truchement d'un routeur WIFI, R.

Le dispositif D peut comporter plusieurs capteurs. Plusieurs dispositifs et/ou plusieurs capteurs peuvent être ainsi disposé de sorte à capturer des flux de données relatifs au bébé.

L'invention permet donc une analyse basée sur des données complémentaires et multimodales, ce qui la rend plus robuste que les techniques connues de surveillance de la qualité du sommeil d'un bébé.

De plus l'utilisation de paramètres physiologiques estimés sans contact permet de fournir une mesure plus précise que les systèmes basés simplement sur l'agitation perceptible (son ou mouvement), tout en restant très simples à utiliser et non-intrusif et gênant pour le bébé.

Bien entendu, la présente invention n'est pas limitée aux exemples et au mode de réalisation décrits et représentés, mais elle est susceptible de nombreuses variantes accessibles à l'homme de l'art.

## Revendications

1. Procédé de détermination automatique d'une information (IQ) représentative de la qualité du sommeil d'un être vivant, sans contact avec ledit être vivant, à partir d'un ensemble de flux de données (F₁, F₂, F₃... F_{N}) relatives audit être vivant, issus d'au moins deux capteurs (C₁, C₂, C₃ ... C_{M}), au moins un desdits capteurs étant une caméra vidéo filmant ledit être vivant et fournissant un flux correspondant à un paramètre physiologique dudit être vivant, établi par photopléthysmographie, ledit procédé consistant à
- établir des flux de mesures à partir desdits flux de données ;
- décomposer le flux établi par photopléthysmographie en régions d'intérêt, et établir une somme pondérée par une pulsatilité mesurée pour chaque région, des signaux de photopléthysmographie établi pour chaque région ;
- combiner lesdits flux de mesures pour fournir ladite information, en en prenant en compte une estimation de la qualité desdits flux de données.

2. Procédé selon la revendication précédente, dans lequel ledit flux de mesures établi par photopléthysmographie est un rythme cardiaque.

3. Procédé selon la revendication précédente, dans lequel ledit flux de mesures établi par photopléthysmographie est un rythme respiratoire.

4. Procédé selon l'une des revendications précédentes, dans lequel lesdits flux de mesures représentent des données environnementales, des données liées à l'activité dudit être vivant, et des données physiologiques.

5. Procédé selon la revendication 2, dans lequel la mesure cardiaque réalisée par photopléthysmographie est améliorée par l'utilisation d'un modèle de la forme du signal PPG comme information a priori pour aider la convergence de la technique de séparation de sources.

6. Procédé selon l'une des revendications 2 à 5, dans lequel un flux de mesures est un rythme respiratoire établi par analyse des mouvements de la cage thoracique dudit être vivant à partir d'un flux vidéo généré par ladite caméra vidéo.

7. Procédé selon l'une des revendications précédentes, dans lequel la combinaison des flux de mesures est réalisée en estimant la fiabilité des flux de mesures par statistique bayésienne.

8. Dispositif (D) pour détermination automatique d'une information (IQ) représentative de la qualité du sommeil d'un être vivant, sans contact avec ledit être vivant, à partir d'un ensemble de flux de données (F₁, F₂, F₃... F_{N}) relatives audit être vivant, issus d'au moins deux capteurs (C₁, C₂, C₃ ... C_{M}), au moins un desdits capteurs étant une caméra vidéo filmant ledit être vivant et fournissant un flux correspondant à une donnée physiologique dudit être vivant, établi par photopléthysmographie, ledit dispositif comportant des moyens pour :
- établir des flux de mesures à partir desdits flux de données ;
- décomposer le flux établi par photopléthysmographie en régions d'intérêt, et établir une somme pondérée par une pulsatilité mesurée pour chaque région, des signaux de photopléthysmographie établi pour chaque région
- combiner lesdits flux de mesures pour fournir ladite information, en en prenant en compte une estimation de la qualité desdits flux de données.

9. Système portant un dispositif selon la revendication précédente, et une caméra vidéo pour filmer ledit être vivant, dans lequel ledit dispositif est prévu pour recevoir au moins un flux de données issu de ladite caméra vidéo.

## Patentansprüche

1. Verfahren zum automatischen Bestimmen einer Information (IQ), die die Schlafqualität eines Lebewesens darstellt, ohne Kontakt mit dem Lebewesen, aus einer Gesamtheit von Datenströmen (F₁, F₂, F₃... F_{N}), die sich auf das Lebewesen beziehen, die von mindestens zwei Sensoren (C₁, C₂, C₃... C_{M}) stammen, wobei mindestens einer der Sensoren eine Videokamera ist, die das Lebewesen filmt und einen Strom bereitstellt, der einem physiologischen Parameter des Lebewesens entspricht, der durch Photoplethysmographie erstellt wird, wobei das Verfahren besteht aus:
- Erstellen von Messströmen aus den Datenströmen;
- Zerlegen des Messstroms, der durch Photoplethysmographie erstellt wird, in interessierende Regionen und Erstellen einer gewichteten Summe durch eine Pulsatilität, die für jede Region gemessen wird, wobei Photoplethysmographiesignale für jede Region erstellt werden;
- Kombinieren der Messströme zum Bereitstellen der Informationen, wobei eine Schätzung der Qualität der Datenströme berücksichtigt wird.

2. Verfahren nach dem vorstehenden Anspruch, wobei der Messstrom, der durch Photoplethysmographie erstellt wird, ein Herzrhythmus ist.

3. Verfahren nach dem vorstehenden Anspruch, wobei der Messstrom, der durch Photoplethysmographie erstellt wird, ein Atemrhythmus ist.

4. Verfahren nach einem der vorstehenden Ansprüche, wobei die Messströme Umweltdaten, Daten in Zusammenhang mit der Aktivität des Lebewesens, und physiologische Daten darstellen.

5. Verfahren nach Anspruch 2, wobei die Herzmessung, die durch Photoplethysmographie durchgeführt wird, durch die Verwendung eines Modells der Form des PPG-Signals als A-priori-Informationen zum Unterstützen der Konvergenz der Quellentrennungstechnik verbessert wird.

6. Verfahren nach einem der Ansprüche 2 bis 5, wobei ein Messstrom ein Atemrhythmus ist, der durch Analyse der Bewegungen des Brustkorbs des Lebewesens aus einem Videostrom erstellt wird, der durch die Videokamera erzeugt wird.

7. Verfahren nach einem der vorstehenden Ansprüche, wobei die Kombination der Messströme durch Schätzen der Zuverlässigkeit der Messströme mittels Bayes-Statistik erfolgt.

8. Vorrichtung (D) zum automatischen Bestimmen einer Information (IQ), die die Schlafqualität eines Lebewesens darstellt, ohne Kontakt mit dem Lebewesen, aus einer Gesamtheit von Datenströmen (F₁, F₂, F₃... F_{N}), die sich auf das Lebewesen beziehen, die von mindestens zwei Sensoren (C₁, C₂, C₃... C_{M}) stammen, wobei mindestens einer der Sensoren eine Videokamera ist, die das Lebewesen filmt und einen Strom bereitstellt, der einem physiologischen Datenelement des Lebewesens entspricht, das durch Photoplethysmographie erstellt wird, wobei die Vorrichtung Mittel aufweist zum:
- Erstellen von Messströmen aus den Datenströmen;
- Zerlegen des Stroms, der durch Photoplethysmographie erstellt wird, in interessierende Regionen und Erstellen einer gewichteten Summe durch eine Pulsatilität, die für jede Region gemessen wird, wobei Photoplethysmographiesignale für jede Region erstellt werden
- Kombinieren der Messströme zum Bereitstellen der Informationen, wobei eine Schätzung der Qualität der Datenströme berücksichtigt wird.

9. System, das eine Vorrichtung nach dem vorstehenden Anspruch trägt, und eine Videokamera zum Filmen des Lebewesens, wobei die Vorrichtung zum Empfangen mindestens eines Datenstroms vorgesehen ist, der von der Videokamera stammt.

## Claims

1. Method for automatically determining information (IQ) representing the quality of sleep of a living being, without contact with said living being, on the basis of a set of data streams (F₁, F₂, F₃... F_{N}) relating to said living being, the data streams coming from at least two sensors (C₁, C₂, C₃...C_{M}), at least one of the sensors being a video camera which films said living being and provides a stream corresponding to a physiological parameter of said living being, which is established by photo plethysmography, the method consisting in
- establishing measurement streams from said data streams;
- decomposing the stream established by photoplethysmography into regions of interest and establishing a sum, which is weighted according to a pulsatility measured for each region, of the photoplethysmography signals established for each region;
- combining the measurement streams to provide said information, while taking into account an estimate of the quality of the data streams.

2. Method according to the preceding claim, wherein the measurement stream established by photoplethysmography is a heart rate.

3. Method according to the preceding claim, wherein the measurement stream established by photoplethysmography is a respiratory rate.

4. Method according to one of the preceding claims, wherein the measurement streams represent environmental data, data related to the activity of said living being, and physiological data.

5. Method according to claim 2, wherein the cardiac measurement carried out by photoplethysmography is improved by using a model of the shape of the PPG signal as a priori information in order to help the convergence of the source separation technique.

6. Method according to one of claims 2 to 5, wherein a measurement stream is a respiratory rate which is established by analyzing the movements of the rib cage of said living being from a video stream generated by said video camera.

7. Method according to one of the preceding claims, wherein the measurement streams are combined according to an estimate of the reliability of the measurement streams using Bayesian statistics.

8. Device (D) for automatically determining information (IQ) representing the quality of the sleep of a living being, without contact with said living being, on the basis of a set of data streams (F₁, F₂, F₃... F_{N}) relating to said living being, the data streams coming from at least two sensors (C₁, C₂, C₃...C_{M}), at least one of the sensors being a video camera which films said living being and provides a stream corresponding to a piece of physiological data relating to said living being, which is established by photoplethysmography, said device comprising means for:
- establishing measurement streams from said data streams;
- decomposing the stream established by photoplethysmography into regions of interest and establishing a sum, which is weighted according to a pulsatility measured for each region, of the photoplethysmography signals established for each region
- combining the measurement streams to provide said information, while taking into account an estimate of the quality of the data streams.

9. System having a device according to the preceding claim and a video camera for filming said living being, wherein the device is provided for receiving at least one data stream from said video camera.
